# EUROPEAN PATENT APPLICATION

(11) **EP 4 710 780 A1**
(43) Date of publication of application: **18.03.2026**
(21) Application number: 24806272.1
(22) Date of filing: 17.04.2024
(51) Int. Cl.: A24F 40/20, A24F 40/42, A24F 40/85, A61M 15/00

(54) **AEROSOL GENERATING SYSTEM, AEROSOL GENERATING SUBSTRATE ASSEMBLY, AND AEROSOL GENERATING SUBSTRATE**

(30) Priority: 12.05.2023 CN 202310540089
(71) Applicant: Smoore International Holdings Limited, Grand Cayman, KY1-1111 (KY)
(72) Inventor: ZHENG, Wei, Shenzhen, Guangdong 518102 (CN); XIONG, Wei, Shenzhen, Guangdong 518102 (CN); LUO, Yongjie, Shenzhen, Guangdong 518102 (CN)
(74) Representative: Winter, Brandl - Partnerschaft mbB
(86) International application number: PCT/CN2024/088447
(87) International publication number: WO 2024/234915

(57) **Abstract**

The present disclosure relates to an aerosol generating system, an aerosol generating substrate assembly, and an aerosol generating substrate. The aerosol generating substrate includes: a base band; and at least two medium layers formed on the base band and arranged at intervals in a length direction of the base band to generate aerosols through heating, where each medium layer is configured to be capable of being separated from the base band after being heated. According to the aerosol generating substrate, the at least two medium layers are formed on the base band, the at least two medium layers are arranged at intervals in the length direction of the base band, and each medium layer is configured to be capable of being separated from the base band after being heated, thereby preventing the phenomenon that the medium layers harden after being heated and atomized, such that the aerosol generating substrate is blocked in a transmission process, improving smoothness of the aerosol generating substrate in the transmission process, and improving user experience.

## Description

### TECHNICAL FIELD

The present disclosure relates to the field of atomization, and in particular to an aerosol generating system, an aerosol generating substrate assembly, and an aerosol generating substrate.

### BACKGROUND

In the related technologies, an aerosol generating device is capable of serving to heat a cylindrical aerosol generating substrate to generate an aerosol. Atomization media before and after the aerosol generating substrate is atomized are blended together, which easily blends smell of the aerosol, and affects inhaling experience of a user. In some related technologies, the aerosol generating substrate can be made into a band shape to solve the above problem. How to ensure smooth transmission of the aerosol generating substrate is another key issue. Specifically, the atomization media further harden after the aerosol generating substrate is atomized, resulting in transmission blockage.

### SUMMARY

The technical problem to be solved by the present disclosure is to provide an improved aerosol generating substrate, and further provide an improved aerosol generating substrate assembly and aerosol generating system.

The technical solution used in the present disclosure to solve the technical problem of the present disclosure is as follows: an aerosol generating substrate is constructed, and includes:
a base band; and
at least two medium layers formed on the base band and arranged at intervals in a length direction of the base band to generate aerosols through heating, where each medium layer is configured to be capable of being separated from the base band after being heated.

In some embodiments, the medium layers are formed by applying atomization media to the base band.

In some embodiments, each medium layer is sheet-like.

In some embodiments, two medium layers arranged adjacent to each other extend by the same length in a length direction of the base band.

In some embodiments, the number of the at least two medium layers is greater than two; and all the medium layers are equidistantly distributed.

In some embodiments, a non-atomization region is formed in a region that is located between two adjacent medium layers on the base band and has no atomization medium.

In some embodiments, the base band includes a metal sheet or a metal mesh.

An aerosol generating substrate assembly is further constructed in the present disclosure. The aerosol generating substrate assembly includes the aerosol generating substrate of the present disclosure, a first accommodating structure accommodating the aerosol generating substrate to be heated, and a second accommodating structure accommodating a base band of the atomized aerosol generating substrate.

An aerosol generating system is further constructed in the present disclosure. The aerosol generating system includes an aerosol generating device, and the aerosol generating substrate assembly of the present disclosure that is mounted on the aerosol generating device.

In some embodiments, an aerosol generating substrate assembly includes an atomization housing, where
an atomization cavity is formed on an inner side of the atomization housing;
an inlet for allowing an aerosol generating substrate to be heated to enter an atomization cavity is provided on the atomization housing;
an outlet for allowing an atomized aerosol generating substrate to be conveyed out is provided on the atomization housing; and
medium layers of the aerosol generating substrate are separated from a base band on one side of the outlet away from the inlet.

The aerosol generating system, the aerosol generating substrate assembly, and the aerosol generating substrate of the present disclosure are implemented, and have the following beneficial effects: according to the aerosol generating substrate, the at least two medium layers are formed on the base band, the at least two medium layers are arranged at intervals in the length direction of the base band, and each medium layer is configured to be capable of being separated from the base band after being heated, thereby preventing the phenomenon that the medium layers harden after being heated and atomized, such that the aerosol generating substrate is blocked in a transmission process, improving smoothness of the aerosol generating substrate in the transmission process, and improving user experience.

### BRIEF DESCRIPTION OF THE DRAWINGS

The present disclosure will be further described below in combination with the accompanying drawings and the embodiments. In the figures:
FIG. 1 is a schematic structural diagram of an aerosol generating system according to some embodiments of the present disclosure;
FIG. 2 is a cutaway view of the aerosol generating system shown in FIG. 1;
FIG. 3 is a schematic exploded view of a local structure of the aerosol generating system shown in FIG. 1;
FIG. 4 is a schematic structural diagram of an aerosol generating substrate assembly in the aerosol generating system shown in FIG. 3;
FIG. 5 is a schematic exploded view of a local structure of the aerosol generating substrate assembly shown in FIG. 4;
FIG. 6 is a schematic diagram of a local structure of the aerosol generating substrate assembly shown in FIG. 5;
FIG. 7 is a schematic structural diagram of an aerosol generating substrate in the aerosol generating substrate assembly shown in FIG. 6;
FIG. 8 is a schematic structural diagram of an atomization housing in the aerosol generating substrate assembly shown in FIG. 6;
FIG. 9 is a schematic structural diagram of an aerosol generating device in the aerosol generating system shown in FIG. 3; and
FIG. 10 is a schematic exploded view of a local structure of the aerosol generating device in the aerosol generating system shown in FIG. 9.

Description of the reference numerals: 100, aerosol generating system; 10, aerosol generating substrate assembly; 11, box body; 12, aerosol generating substrate; 121, base band; 121a, first surface; 121b, second surface; 1210, non-atomization region; 122, medium layer; 13, first accommodating structure; 131, first accommodating wheel; 1311, first disk body; 1312, second disk body, 14, second accommodating structure; 141, second accommodating wheel; 1411, first disk body; 1412, second disk body; 15, atomization housing; 15a, body portion; 15b, extension portion; 151, atomization cavity; 152, inlet; 153, outlet; 16, guide structure; 161, first guide roller; 162, second guide roller; 163, third guide roller; 164, fourth guide roller; 20, aerosol generating device; 21, machine body; 211, enclosure; 211a, first housing; 211b, second housing; 2111, accommodating cavity; 2112, assembly port; 212, cover body; 22, heating structure; 221, base; 222, heating element; 23, driving assembly; 24, position detection structure; 25, power supply; and 26, main control board.

### DETAILED DESCRIPTION

In order to understand the technical features, objective, and effects of the present disclosure more clearly, specific implementations of the present disclosure are now described in detail with reference to the accompanying drawings. In the following description, it should be understood that the terms "front", "upper", "lower", "top", "bottom", "inner", "outer", etc. indicate azimuthal or positional relationships based on those shown in the accompanying drawings and are constructed and operative in a particular orientation only for ease of description of the technical solution, are not intended to indicate that the device or element indicated needs to have a particular orientation, and thus cannot be construed as a limitation on the present disclosure.

It should be further noted that unless explicitly specified and defined otherwise, the terms "mount", "connect", "arrange", etc. should be understood in a broad sense. For instance, they can denote fixed connection, detachable connection, or integral connection, denote mechanical connection, or electric connection, denote direct connection or indirect connection through an intermediate medium, or denote communication between interiors of two elements or interaction between two elements. When an element is referred to as being "above" or "below" another element, the element can be "directly" or "indirectly" located above the another element, or one or more intervening elements may exist. The terms "first", "second", "third", etc. are only for the convenience of describing the technical solution, and cannot be understood as indicating or implying relative importance or implicitly specifying the number of technical features indicated. Thus, the features limited by "first", "second", "third", etc. can explicitly or implicitly include one or more of the features. Those of ordinary skill in the art can understand the specific meanings of the above terms in the present disclosure according to specific situations.

In the following description, for the purpose of description rather than limitation, specific details such as the specific system structure and technology are provided to thoroughly understand the embodiments of the present disclosure. However, those skilled in the art should be aware that the present disclosure may alternatively be implemented in other embodiments without these specific details. In other cases, detailed descriptions of well-known systems, devices, circuits, and methods are omitted to prevent unnecessary details from hindering the description of the present disclosure.

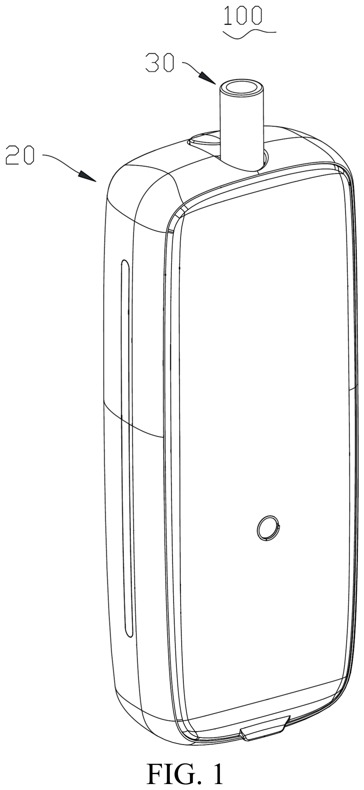

FIG. 1 shows some embodiments of an aerosol generating system of the present disclosure. The aerosol generating system 100 may generate an aerosol for a user to inhale. The aerosol generated by the aerosol generating system 100 has the advantages of excellent taste and excellent user experience.

The aerosol generating system 100 includes an aerosol generating substrate assembly 10 and an aerosol generating device 20. The aerosol generating substrate assembly 10 may be mounted on the aerosol generating device 20, and is used for generating the aerosol when heated. The aerosol generating substrate assembly 10 may be detachably assembled to the aerosol generating device 20, thereby replacing the aerosol generating substrate assembly 10 conveniently. The aerosol generating device 20 may generate and output the aerosol by heating an aerosol generating substrate 12 in the aerosol generating substrate assembly 10 to atomize the aerosol generating substrate.

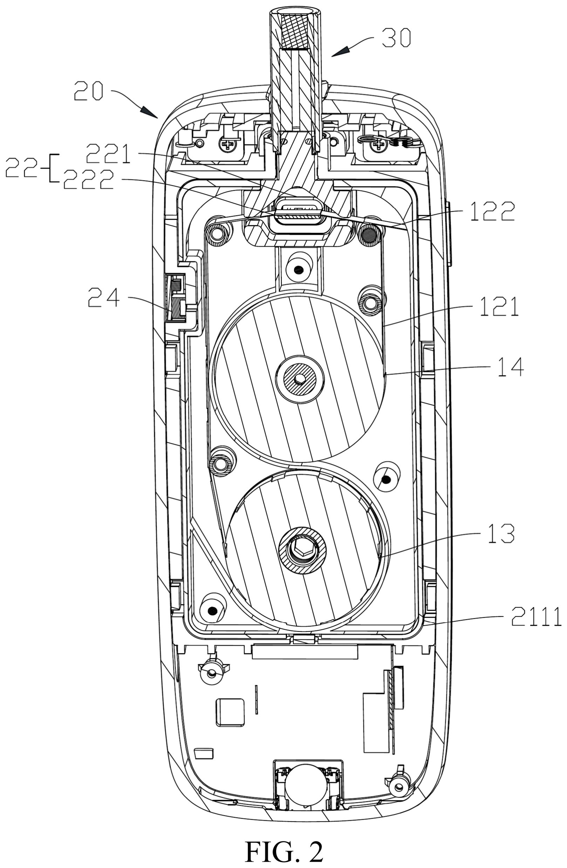

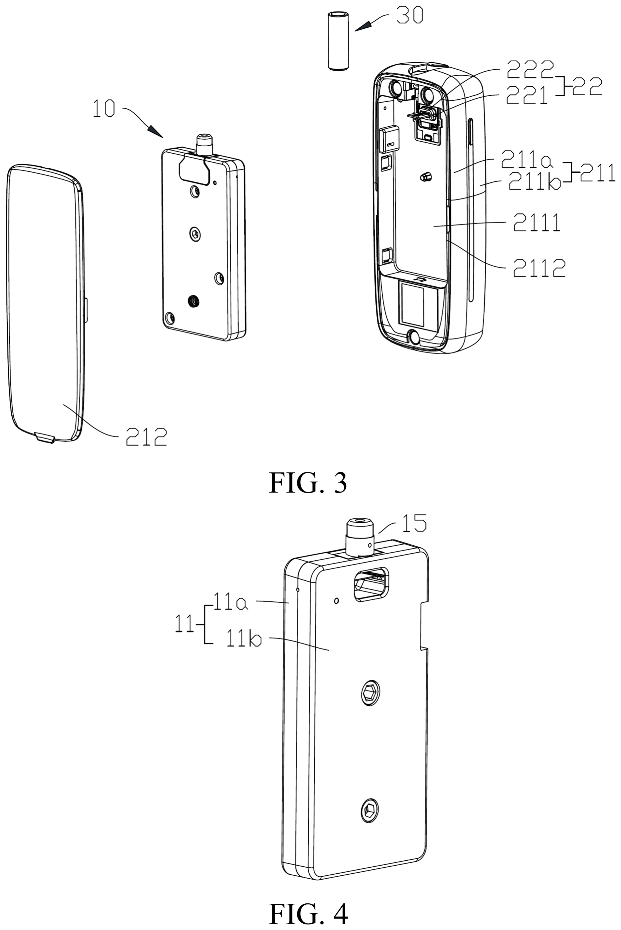

As shown in FIG. 2 to FIG. 4, in some embodiments, an aerosol generating substrate assembly 10 includes a box body 11 and an aerosol generating substrate 12. The box body 11 is used for accommodating the aerosol generating substrate 12. The aerosol generating substrate 12 may be atomized to generate an aerosol in a heated state. Understandably, in some other embodiments, a box body 11 may be omitted. An aerosol generating substrate 12 may be directly mounted in an aerosol generating device 20.

In some embodiments, a box body 11 may be of a transparent structure, and may be a transparent plastic box, for example. Clearly, understandably, in some other embodiments, a box body 11 is not limited to a transparent structure, and may be a non-transparent structure, for example, a non-transparent plastic box or a non-transparent metal box. In some embodiments, a box body 11 may include a first box body 11a and a second box body 11b. Shapes and sizes of the first box body 11a and the second box body 11b are equivalent. In some embodiments, a first box body 11a and a second box body 11b are approximately cuboid-shaped. The first box body 11a and the second box body 11b may not be limited to being cuboid-shaped, for example, cylindrically cube-shaped or irregular. A cavity is formed on an inner side of each of the first box body 11a and the second box body 11b, and the first box body and the second box body are open structures. One side of the first box body 11a having a splicing port may be spliced to one side of the second box body 11b having a splicing port. The first box body 11a and the second box body 11b may be connected and fixed by arranging a connecting structure. The connecting structure may be a screwing assembly, a clamping assembly, a hinge structure, or any other type. In some embodiments, a connecting structure may alternatively be omitted. A first box body 11a and a second box body 11b may be connected by using a conventional ultrasonic technology.

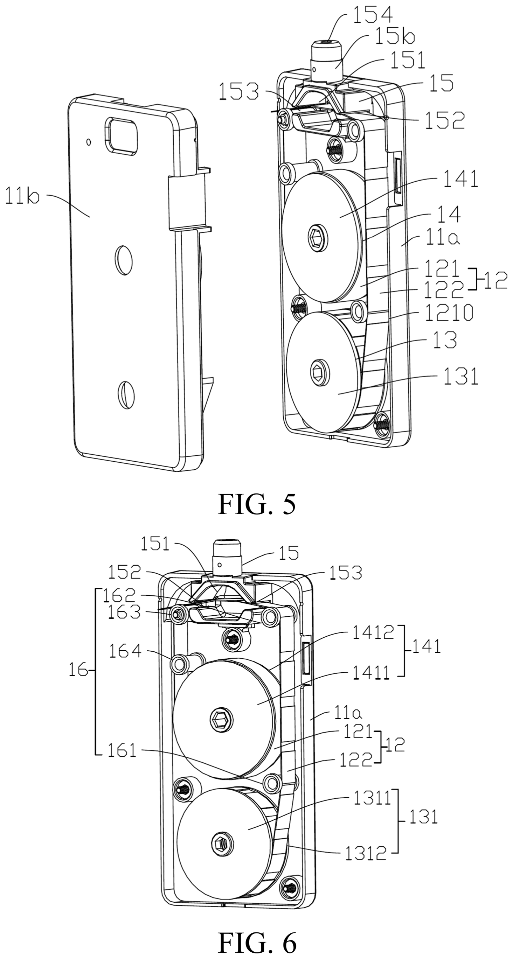

As shown in FIG. 5 to FIG. 7, in some embodiments, an aerosol generating substrate 12 is banded in a whole, and may be movably wound. In some embodiments, an aerosol generating substrate 12 includes a base band 121 and medium layers 122. The base band 121 is used for carrying the medium layers 122. The medium layers 122 are formed on the base band 121. Specifically, the medium layers 122 may be formed by applying atomization media to the base band 121. The atomization media may be a liquid and curable atomization medium, or may be a pasty atomization medium or a solid atomization medium. In some other embodiments, medium layers 122 are not limited to being formed by applying atomization media, and may alternatively be formed by pressing or pasting atomization media on a base band 121, for example.

In some embodiments, a base band 121 is longitudinally arranged, and has a first surface 121a and a second surface 121b arranged opposite to each other. The first surface 121a and the second surface 121b may be surfaces defined by a long edge and a wide edge of the base band 121. The first surface 121a may carry the medium layers 122. In some embodiments, a base band 121 may be an easily heat-conducting metal sheet, for example, an aluminum foil or a copper foil. Clearly, in some other embodiments, a base band 121 is not limited to a metal sheet, and may alternatively be a metal mesh. The metal mesh may be formed by braiding a metal wire, or may be formed by providing a plurality of through holes on the metal sheet. Clearly, the base band 121 is not limited to being made of metal, and may alternatively be made of other materials having high thermal conductivity or an excellent soaking effect, such as graphite, silicon carbide, or other surface treated materials. This is not limited in the present application. The base band 121 is made of a material having high thermal conductivity or an excellent soaking effect, such that the atomization efficiency of media may be higher, and the media may be heated more uniformly.

Specifically, the medium layers 122 may be formed on the first surface 121a of the base band 121. The at least two medium layers 122 may be provided. Specifically, the number of the medium layers 122 may be greater than two. The plurality of medium layers 122 may be arranged at intervals in a length direction of the base band 121. In some embodiments, a plurality of medium layers 122 may be equidistantly arranged in a length direction of a base band 121. That is, the length of each interval is the same. Each medium layer 122 may be approximately a rectangular sheet structure. Understandably, in some other embodiments, medium layers 122 are not limited to being rectangular, and may be elliptical, circular, etc. In some embodiments, two medium layers 122 arranged adjacent to each other extend by the same length in a length direction of a base band 121. That is, the lengths of the medium layers 122 are the same. The two medium layers 122 arranged adjacent to each other extend by the same length in the length direction of the base band 121, and all the medium layers 122 may be equidistantly arranged, thereby conveniently determining a position of an aerosol generating substrate 12 in a transmission process. In some other embodiments, at least two medium layers 122 in all medium layers 122 may alternatively be non-equidistantly distributed. In some other embodiments, at least two medium layers 122 in all medium layers 122 may extend by different lengths in a length direction of a base band 121. In some embodiments, the thicknesses of two medium layers 122 arranged adjacent to each other may be the same, and the two medium layers arranged adjacent to each other may alternatively extend by the same length in a width direction of a base band 121. That is, the medium layers 122 generate the same maximum amount of aerosols when being heated and atomized, and the aerosols may be uniformly inhaled. Clearly, understandably, in some other embodiments, the thicknesses and/or the lengths extending in a width direction of a base band 121 of two medium layers 122 arranged adjacent to each other may alternatively be different.

In some embodiments, a non-atomization region 1210 is formed in a region that is on a base band 121 and arranged corresponding to an interval between two medium layers 122 arranged adjacent to each other. That is, no atomization medium is applied to the region. In some other embodiments, a non-atomization region 1210 may alternatively be formed in a region that is on a base band 121 and arranged corresponding to an interval between two medium layers 122 arranged adjacent to each other by applying a non-atomization material. The thickness of the applied non-atomization material may be less than the thicknesses of the medium layers 122. By forming the non-atomization region 1210, a position detection structure 24 in an aerosol generating device 20 may conveniently determine a position of an aerosol generating substrate 12 according to inconsistency of reflectivity of the medium layers 122 and reflectivity of the non-atomization region 1210, thereby providing an accurate signal for feeding of the aerosol generating substrate 12.

In some embodiments, each medium layer 122 may be configured to be capable of being separated from a base band 121 after being heated, thereby preventing the phenomenon that the medium layers 122 harden after being heated and atomized, such that the aerosol generating substrate 12 is blocked in a transmission process, improving smoothness of the aerosol generating substrate 12 in the transmission process, and improving user experience. After the medium layers 122 are heated and atomized through a heating structure 22, atomization media in the medium layers 122 become hard after being heated, and a pasting effect between the medium layers 122 and the base band 121 is reduced. In a process in which the aerosol generating substrate 12 continues being transmitted, if the medium layers 122 are separated from the base band 121, the base band 121 may be prevented from being blocked to continue to move forwards. Thus, the aerosol generating substrate 12 may continue being transmitted, and transmission is more smooth. In some embodiments, heated medium layers 122 and a base band 121 may be automatically separated, or may be separated through a separation structure. In some embodiments, medium layers 122 may be partially separated from a base band 121, or may be completely separated. That is, the medium layers 122 may be separated from the base band 121 until recovery and transmission of the aerosol generating substrate 12 are not influenced.

In some embodiments, an aerosol generating substrate assembly 10 further includes a first accommodating structure 13. The first accommodating structure 13 may be used for accommodating an aerosol generating substrate 12 to be heated. In some embodiments, a first accommodating structure 13 is arranged in a box body 11. The first accommodating structure 13 may be mounted between a first box body 11a and a second box body 11b, and is connected to the first box body 11a and the second box body 11b. In some embodiments, a first accommodating structure 13 may be a first accommodating wheel 131. An aerosol generating substrate 12 to be heated may be wound around the first accommodating wheel 131, and the first accommodating wheel 131 may drive the aerosol generating substrate 12 to be conveyed through rotation. In some embodiments, a first accommodating wheel 131 includes a first disk body 1311 and a second disk body 1312. The first disk body 1311 and the second disk body 1312 may be approximately circular, and the radial dimensions of the first disk body 1311 and the second disk body 1312 may be approximately equivalent. The first disk body 1311 is connected to the second disk body 1312 through a hollow winding drum. The winding drum may be formed at an axis of the first disk body 1311 and/or the second disk body 1312, or may be arranged separately, and two ends of the winding drum are connected to the first disk body 1311 and the second disk body 1312 respectively. The radial dimension of the winding drum is less than the radial dimensions of the first disk body 1311 and the second disk body 1312, and the aerosol generating substrate 12 to be heated may be wound around the winding drum. Understandably, in some other embodiments, a first disk body 1311 and a second disk body 1312 may not be limited to being circular, and may alternatively be square. The first disk body 1311 and the second disk body 1312 may be used for blocking an aerosol generating substrate 12 from being separated in a winding process. In some embodiments, a first disk body 1311 and a second disk body 1312 may alternatively be omitted. In some embodiments, a first accommodating wheel 131 is rotatably connected to a first box body 11a and/or a second box body 11b, and may drive an aerosol generating substrate 12 to be heated to be wound and unwound through rotation, thereby accommodating and conveying the aerosol generating substrate 12 to be heated.

In some embodiments, an aerosol generating substrate assembly 10 further includes a second accommodating structure 14. The second accommodating structure 14 is used for accommodating a base band 121 of an atomized aerosol generating substrate 12. That is, the second accommodating structure may accommodate the base band 121 from which medium layers 122 are separated. In some embodiments, a second accommodating structure 14 may include a second accommodating wheel 141. A heated base band 121 may be wound around the second accommodating wheel 141. In some embodiments, a second accommodating wheel 141 is arranged between a first box body 11a and a second box body 11b, and the first box body 11a is connected to the second box body 11b. In some embodiments, a second accommodating wheel 141 includes a third disk body 1411 and a fourth disk body 1412. The third disk body 1411 and the fourth disk body 1412 are circular, and the radial dimensions of the third disk body and the fourth disk body are approximately equivalent. The third disk body 1411 and the fourth disk body 1412 are arranged at an interval and are connected through a hollow reel. The reel may be formed at an axis of the third disk body 1411 and/or the fourth disk body 1412, or may be arranged separately, and two ends of the reel are connected to the third disk body 1411 and the fourth disk body 1412 respectively. The reel may be cylindrical, the radial dimension of the reel is less than the radial dimensions of the third disk body 1411 and the fourth disk body 1412, and the heated base band 121 may be wound around the reel. In some embodiments, a third disk body 1411 and a fourth disk body 1412 may not be limited to being circular, and may alternatively be square. The third disk body 141 and the fourth disk body 1412 may block a base band 121 from being separated. In some embodiments, a third disk body 1411 and a fourth disk body 1412 may be omitted. In some embodiments, a second accommodating wheel 141 may be rotatably connected to a first box body 11a and/or a second box body 11b, and may drive a base band 121 to be wound around the second accommodating wheel through rotation.

In some embodiments, a second accommodating wheel 141 may form a driving wheel, and a first accommodating wheel 131 may form a driven wheel. Because a base band 121 is wound around the first accommodating wheel 131 and the second accommodating wheel 142, the first accommodating wheel 131 may rotate under a driving effect of the second accommodating wheel 141. When rotating simultaneously, the first accommodating wheel 131 and the first accommodating wheel 131 drive an aerosol generating substrate 12 to be heated to be fed, and drive a base band of the atomized aerosol generating substrate 12 to be wound around the second accommodating wheel 141. In some other embodiments, a second accommodating wheel 141 is not limited to a driving wheel, and may alternatively be a driven wheel. A first accommodating wheel 131 is not limited to a driven wheel, and may alternatively be a driving wheel. In some embodiments, a first accommodating wheel 131 and a second accommodating wheel 142 may alternatively be rotatably arranged separately. That is, the first accommodating wheel 131 may be driven to rotate by one set of driving structures, and the second accommodating wheel 142 may be driven to rotate by another set of driving structures.

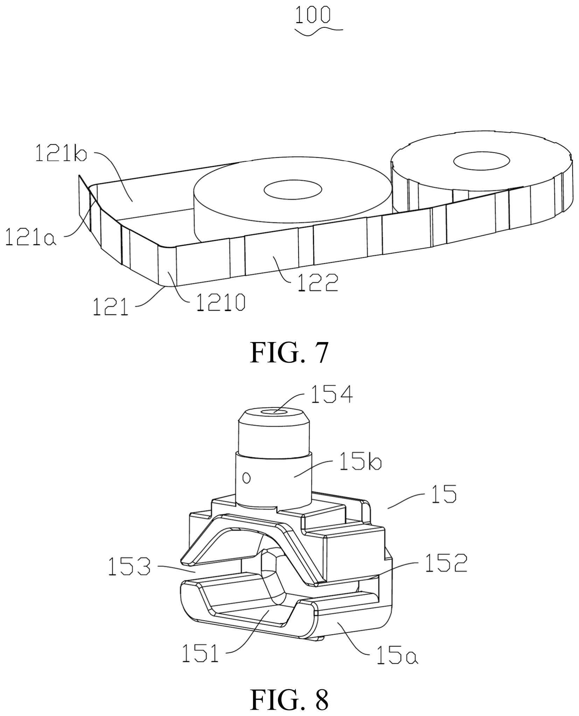

As shown in FIG. 8, in some embodiments, an aerosol generating substrate assembly 10 further includes an atomization housing 15. The atomization housing 15 is partially arranged on a box body 11. The atomization housing 15 includes a body portion 15a and an extension portion 15b arranged on the body portion 15a. The body portion 15a may be embedded into the box body 11, and an atomization cavity 151 is formed on an inner side of the atomization housing 15. An inlet 152 is provided on the atomization housing 15. An outlet 153 is provided on the atomization housing 15. The atomization cavity 151 is formed on an inner side of the body portion 15a, and the atomization cavity 151 is defined by space in which a heating structure 22 heats an aerosol generating substrate 12. The inlet 152 is provided on one side of the body portion 15a, is in communication with the atomization cavity 151, and is used for allowing the aerosol generating substrate 12 to be heated to enter the atomization cavity 151. The outlet 153 is provided on the other side of the body portion 15a, is in communication with the atomization cavity 151, and is used for allowing the atomized aerosol generating substrate 12 to be conveyed out. The inlet 152 and the outlet 153 are provided opposite each other, and are located in or close to a plane in which a center of the atomization cavity 151 is located. The extension portion 15b is arranged on the body portion 15a, may pass out from the box body 11, and is used for connecting to a mouthpiece assembly 30. The extension portion 15b is cylindrical, and is of a two-end through structure, and an air outlet channel 154 in communication with the atomization cavity 151 is formed on an inner side of the extension portion, and is used for allowing an aerosol formed by atomization to be output. In some other embodiments, an inlet 152 and an outlet 153 may alternatively not be limited to being arranged opposite each other. For example, the inlet 152 and the outlet 153 may be located on two side surfaces of an atomization cavity 151 arranged adjacent to each other. In some other embodiments, an atomization cavity 151 may alternatively only have an inlet 152 or an outlet 153.

As further shown in FIG. 5 and FIG. 6, in some embodiments, an aerosol generating substrate assembly 10 further includes a guide structure 16. The guide structure 16 is arranged in a box body 11 and is used for guiding transmission on an aerosol generating substrate 12. In some embodiments, a guide structure 16 includes a first guide roller 161, a second guide roller 162, a third guide roller 163, and a fourth guide roller 164 which are sequentially arranged. The first guide roller 161 is arranged on one side of a first accommodating structure 13. The second guide roller 162 and the third guide roller 163 are arranged on two opposite sides of an atomization housing 15 respectively. The second guide roller 162 is located on one side of an inlet 152 away from an outlet 153, and the third guide roller 163 is located on one side of the outlet 153 away from the inlet 152. The fourth guide roller 164 is located on one side of a second accommodating structure 14. A connecting line between the fourth guide roller 164 and the third guide roller 163 and a connecting line between the second guide roller 162 and the third guide roller 163 are arranged at an included angle, and the included angle is an angle greater than 0 degree and less than 180 degrees. When a first accommodating wheel 131 and a second accommodating wheel 141 rotate, an aerosol generating substrate 12 to be heated may enter an atomization cavity 151 along the first guide roller 161, the second guide roller 162, and the inlet 152. After atomization, the first accommodating wheel 131 and the second accommodating wheel 141 continue rotating, and the atomized aerosol generating substrate 12 may be output from the outlet 153 and pass through the third guide roller 163. When being transmitted to the fourth guide roller 164 through the third guide roller 163, the atomized aerosol generating substrate 12 needs to be turned. Because heated and atomized medium layers 122 become hard, the aerosol generating substrate 12 may be directly separated from a base band 121 at a turning when continuing being conveyed. That is, the aerosol generating substrate directly falls off. Thus, the phenomenon that the hardened medium layers 122 are blocked when passing through a gap between the third guide roller 163 and the box body 11 may be avoided. The base band 121 separated from the medium layers 122 may continue to be accommodated into the second accommodating wheel 141 along the fourth guide roller 164.

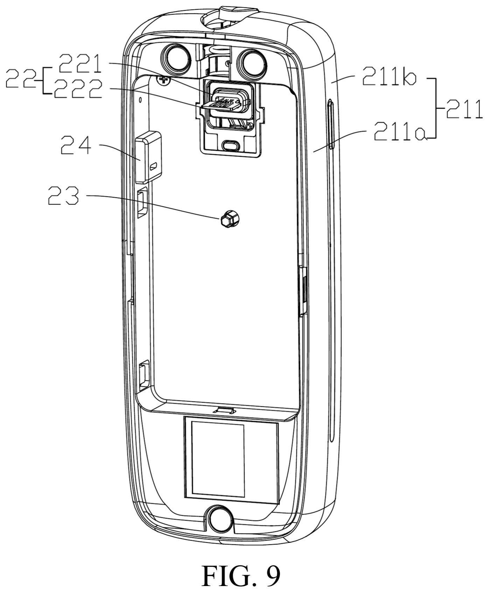

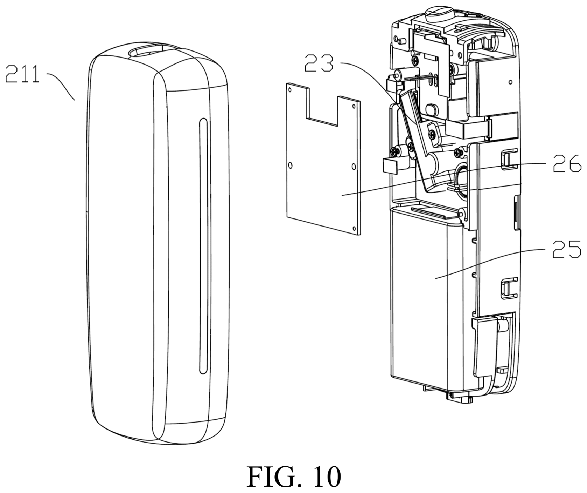

With reference to FIG. 1 to FIG. 3, FIG. 9 and FIG. 10 together, an aerosol generating device 20 includes a machine body 21 and a heating structure 22. The machine body 21 may be used for accommodating an aerosol generating substrate assembly 10. The heating structure 22 is mounted on the machine body 21, may be inserted into the aerosol generating substrate assembly 10, and is used for heating an aerosol generating substrate 12 in the aerosol generating substrate assembly 10 to generate an aerosol.

In some embodiments, a machine body 21 includes an enclosure 211 and a cover body 212. The enclosure 211 includes a first housing 211a and a second housing 211b. The first housing 211a is arranged on the second housing 211b, an accommodating cavity 2111 is provided on the first housing 211a, and the accommodating cavity 2111 is used for accommodating the aerosol generating substrate assembly 10. An assembly port 2112 is provided on a side wall of the first housing 211a, and the assembly port 2112 is used for allowing the aerosol generating substrate to be loaded into the accommodating cavity 2111. The cover body 212 is arranged at the assembly port 2112, and detachably covers the accommodating cavity 2111. By opening the cover body 212, the aerosol generating substrate assembly 10 may be convenient to remove and replace.

In some embodiments, a heating structure 22 is mounted on a first housing 211a, is located on a bottom wall of an accommodating cavity 2111, and protrudes towards the accommodating cavity 2111. When an aerosol generating substrate assembly 10 is mounted in the accommodating cavity 2111, the heating structure 22 may be inserted into an atomization housing 15 of the aerosol generating substrate assembly 10, and is used for heating the aerosol generating substrate 12 to be heated that enters an atomization cavity 151. In some embodiments, a heating structure 22 includes a base 221 and a heating element 222. The base 221 is mounted on a bottom wall of an accommodating cavity 2111, and the heating element 222 is mounted on the base 221. In some embodiments, a heating element 222 includes a metal heating sheet. Clearly, understandably, in some other embodiments, a heating element 222 is not limited to a metal heating sheet.

In some embodiments, an aerosol generating device 20 further includes a driving assembly 23. The driving assembly 23 may be mounted on a machine body 21. Specifically, the driving assembly 23 is mounted between a first housing 211a and a second housing 211b, and the part of the driving assembly may extend into an accommodating cavity 2111 to be connected to a second accommodating structure 14 or a first accommodating structure 13, and is used for transferring an aerosol generating substrate 12 in an aerosol generating substrate assembly 10 to a heating structure 22. In some embodiments, a driving assembly 23 may be an electric driving structure. For example, the driving assembly 23 may be an electric motor or a reduction gearbox. An output shaft of the driving assembly 23 may penetrate the accommodating cavity 2111 from one side of the first housing the first housing 211a arranged opposite to the accommodating cavity 2111, and is connected to the second accommodating structure 14 or the first accommodating structure 13. That is, the output shaft may be connected to a first accommodating wheel 131 or a second accommodating wheel 141, and is used for driving the first accommodating wheel 131 and the second accommodating wheel 132 to rotate. Clearly, understandably, in some other embodiments, a driving assembly 23 may alternatively be not limited to an electric driving structure, and may be a manual driving structure, such as a handle or a hand wheel.

In some embodiments, an aerosol generating device 20 further includes a position detection structure 24. The position detection structure 24 is arranged in an accommodating cavity 2111, and is used for detecting a conveying position of an aerosol generating substrate 12 and feeding back the conveying position to a driving assembly 23 to provide an accurate signal for feeding and running of the driving assembly 23, thereby ensuring precision of movement of the aerosol generating substrate 12. In some embodiments, a position detection structure 24 may be an infrared detection switch. The infrared detection switch is located at a side wall of an accommodating cavity 2111, and is arranged opposite an aerosol generating substrate 12 to be heated. When a driving assembly 23 is started, a first accommodating wheel 131 and a second accommodating wheel 141 may be driven to rotate to drive the aerosol generating substrate 12 to be heated to be fed. When the aerosol generating substrate 12 to be heated moves to the front of an infrared detection switch, the infrared detection switch may determine a position at which the aerosol generating substrate 12 is fed according to reflectivity of medium layers 122 and reflectivity of an non-atomization region 1210. Understandably, in some other embodiments, a position detection structure 24 is not limited to an infrared detection switch. In some other embodiments, a position detection structure 24 may alternatively be a conventional sensor.

In some embodiments, an aerosol generating device 20 further includes a power supply 25. The power supply 25 is arranged in an enclosure 211, and is specifically located between a first housing 211a and a second housing 211b. The power supply 25 may be used for supplying power to a driving assembly 23 and a heating structure 22. In some embodiments, a power supply 25 may be a battery.

In some embodiments, an aerosol generating device 20 further includes a main control board 26. The main control board 26 is arranged in an enclosure 211, and is specifically located between a first housing 211a and a second housing 211b. The main control board 26 is connected to a power supply 25, a driving assembly 23, and a heating structure 22. The main control board 26 is further connected to a position detection structure 24. The main control board 26 may output a startup or shutdown instruction to the driving assembly 23 according to position information of an aerosol generating substrate 12 detected by the position detection structure 24.

In some embodiments, an aerosol generating system further includes a mouthpiece assembly 30. The mouthpiece assembly 30 may be assembled on an atomization housing 15. Specifically, the mouthpiece assembly 30 may sleeve an extension portion 15b, and a user allows a user to inhale an aerosol output from an atomization cavity 151. In some embodiments, a mouthpiece assembly 30 may be approximately cylindrical. Clearly, understandably, in some other embodiments, a mouthpiece assembly 30 is not limited to being cylindrical, and may alternatively be flat cylindrical or other shapes.

Understandably, the above embodiments only describe the implementations of the present disclosure specifically and in detail, but cannot be construed as a limitation on the scope of patent of the present disclosure accordingly. It should be pointed out that for those of ordinary skill in the art, without departing from the concept of the present disclosure, the above technical features can be freely combined, and several deformations and improvements can be further made, all of which fall within the scope of protection of the present disclosure. Thus, any equivalent transformations and modifications made to the scope of the claims of the present disclosure shall fall within the scope of the claims of the present disclosure.

## Claims

1. An aerosol generating substrate, comprising: a base band (121); and at least two medium layers (122) formed on the base band (121) and arranged at intervals in a length direction of the base band (121) to generate aerosols through heating, wherein each medium layer (122) is configured to be capable of being separated from the base band (121) after being heated.

2. The aerosol generating substrate of claim 1, wherein the medium layers (122) are formed by applying atomization media to the base band (121).

3. The aerosol generating substrate of claim 1, wherein each medium layer (122) is sheet-like.

4. The aerosol generating substrate of claim 3, wherein two medium layers (122) arranged adjacent to each other extend by the same length in a length direction of the base band (121).

5. The aerosol generating substrate of claim 1, wherein the number of the at least two medium layers (122) is greater than two; and all the medium layers (122) are equidistantly distributed.

6. The aerosol generating substrate of claim 1, wherein a non-atomization region (1210) is formed in a region that is located between two adjacent medium layers (122) on the base band (121) and has no atomization medium.

7. The aerosol generating substrate of claim 1, wherein the base band (121) comprises a metal sheet or a metal mesh.

8. An aerosol generating substrate assembly, comprising: the aerosol generating substrate (12) of any one of claims 1 to 7, a first accommodating structure (13) accommodating the aerosol generating substrate (12) to be heated, and a second accommodating structure (14) accommodating a base band (121) of the atomized aerosol generating substrate (12).

9. An aerosol generating system, comprising: an aerosol generating device (20), and the aerosol generating substrate assembly (10) of claim 8 that is mounted on the aerosol generating device (20).

10. The aerosol generating system of claim 9, wherein the aerosol generating substrate assembly (10) comprises an atomization housing (15), wherein an atomization cavity (151) is formed on an inner side of the atomization housing (15); an inlet (152) for allowing an aerosol generating substrate (12) to be heated to enter an atomization cavity (151) is provided on the atomization housing (15); an outlet (153) for allowing an atomized aerosol generating substrate (12) to be conveyed out is provided on the atomization housing (15); and medium layers (122) of the aerosol generating substrate (12) are separated from a base band (121) on one side of the outlet (153) away from the inlet (152).
